# EUROPÄISCHE PATENTSCHRIFT

(11) **EP 1 790 965 B1**
(45) Veröffentlichungstag und Bekanntmachung des Hinweises auf die Patenterteilung: **28.11.2007**
(21) Anmeldenummer: 05111435.3
(22) Anmeldetag: 29.11.2005
(51) Int. Cl.: G01N 1/00, G01N 33/28, G01N 1/22

(54) **Verfahren zur Bereitstellung der in Flüssigkeiten von Hochspannungsanlagen gelösten Gase für die externe Analyse und eine Vorrichtung zur Durchführung des Verfahrens**
Method for providing the gases dissolved in liquids from high-voltage systems for external analysis and device for carrying out this method
Procédé de mise à disposition de gaz libérés dans des liquides d'installations à haute tension pour l'analyse externe et dispositif pour la réalisation du procédé

(43) Veröffentlichungstag der Anmeldung: 30.05.2007
(73) Patentinhaber: Bräsel, Eckhard, 17493 Greifswald (DE)
(72) Erfinder: Sasum, Ute Dr., 17491, Greifswald (DE); Roßberg, Harald Dr., 17498, Neuenkirchen (DE)
(74) Vertreter: Garrels, Sabine

(56) Entgegenhaltungen:
- GB-A- 214 548
- US-A- 4 763 514
- SKETCHLEY J M: "Gas in oil analysis-methods and equipment re-assessed" IEE COLLOQUIUM ON "CONDITION MONITORING AND REMANENT LIFE ASSESSMENT IN POWER TRANSFORMERS" (DIGEST NO.1994/075), 1994, IEE, LONDON, UK, 1994, Seiten 5-1, XP006527155

## Beschreibung

### Technisches Gebiet

Die Erfindung betrifft ein Verfahren zur Bereitstellung der in Flüssigkeiten von Hochspannungsanlagen gelösten Gase für die externe Analyse, welches auf Basis des Henry-Dalton'schen Gesetzes entweder der Bestimmung der in der Flüssigkeit gelösten Gase oder deren Löslichkeitskoeffizienten dient. Damit sind die Fehlerfrüherkennung und die Vermeidung von Folgefehlern sowie die Bewertung der Gaslöslichkeit von Flüssigkeiten möglich. Die Erfindung betrifft darüber hinaus eine Vorrichtung zur Durchführung des Verfahrens, die in situ bzw. auch zusätzlich als Transportgefäß bis ins Labor und als Aufbewahrungsgefäß eingesetzt werden kann.

### Stand der Technik

Hochspannungsanlagen, zu denen auch die Leistungstransformatoren gehören, werden mit Flüssigkeiten, insbesondere mit Mineralölen, als Dielektrikum und Kühlmittel gefüllt. Darüber hinaus hat sich das Betriebsöl auch als Diagnosemedium bewährt. Anormale elektrische und thermische Belastungen des Öl- / Zellulose-Isolationssystems führen zur Bildung von fehlerartspezifischen Fehlergasen, die sich bei schleichender Entwicklung lange Zeit im Öl auflösen bevor sie bei Gassättigung des Öles das Gassammelrelais erreichen oder einen Folgefehler verursachen können. Für die Zustandsdiagnostik von Transformatoren ist es erforderlich, über die Überwachung der Fehlergase hinaus auch die atmosphärischen Gase zu überwachen. Nur so können die Zusammenhänge des Gashaushaltes die Zuverlässigkeit der Diagnostik steigern. Die Überwachung entwickelt sich von Laborverfahren hin auch zu In Situ- und Online-Verfahren (Bräsel, E.; Sasum, U., Gesamtanalytische Transformatorendiagnostik - die Entwicklung von der Labor- zur Onlineüberwachung, VGB - Konferenz "Chemie im Kraftwerk 2004" 27./28.10.2004 Essen). In der internationalen Norm DIN IEC 60 567 ("Anleitung für die Probenahme von Gasen und von Öl aus ölgefilterten elektrischen Betriebsmitteln und für die Analyse freier und gelöster Gase") sind gebräuchliche Laborverfahren der Vakuumextraktion, des Strippings sowie Headspace-Varianten beschrieben.

Laborvergleiche zeigen, dass es bei der Reproduzierbarkeit und Richtigkeit der Messergebnisse erhebliche Abweichungen geben kann. Als Ursache müssen die vielen Einzelschritte bis zur eigentlichen gaschromatographischen Analyse angesehen werden: Probenahme am Transformator (Ablaufmenge), Probegefäße, Transport und Aufbewahrung auf der einen Seite und auf der anderen Seite die Gasextraktion.

Bei der Vakuumextraktion können Undichtigkeiten auftreten. Beim Stripping bieten die kleinen Volumina Fehlerangriffspunkte, ähnlich wie bei der Headspace-Gaschromatographie. Die Vakuumextraktion wird auch online sowie in situ eingesetzt. Zusätzlich existieren Membranverfahren, die den Gasdurchtritt aus dem Öl in den Analysenraum gewährleisten. Ihr gemeinsamer Nachteil ist, dass sie in der Regel keine aktive Ölbewegung ermöglichen, so dass die Ergebnisse nicht immer repräsentativ sind.

Als Headspace-Varianten können alle Verfahren angesehen werden, die eine Ölprobe mit einem gefüllten Gasraum in Verbindung bringen und eine Kontaktierung bis zum Gleichgewichtszustand stattfindet. Über die Gasraumanalyse wird dann nach dem Henry-Dalton'schen Gesetz der Gasgehalt der Ölprobe bestimmt. Wird das Volumen der Ölprobe viel größer als das Gasraumvolumen gewählt, erhält man im Gleichgewichtszustand den Lösungsdruck der Ölprobe im Gasraum. Das Ölvolumen kann durch kontinuierliches Strömen vergrößert werden (DE 101 22 173 C2) oder durch periodische Wiederholung der Kontaktierung mit immer wieder neuem Öl (DE 198 33 601 C1). Das Ausgangsfüllgas ist im einfachsten Fall Luft, die Gasart spielt aber keine Rolle. Diese Verfahren werden online gestaltet.

Als In Situ-Verfahren ist der Shake-Test in der DIN IEC 60567 aufgeführt. Nach dem Shake-Test wird eine in einer Spritze befindliche Ölprobe mit einem Luftvolumen in der Endlage des Kolbens geschüttelt. Nach einer vorgegebenen Schütteldauer wird die Endlage des Kolbens wieder hergestellt und die Spritze kurzzeitig belüftet und wieder geschüttelt. Das wird nochmals wiederholt und dann die Gasdosierung in den Analysator vorgenommen. Wegen der Luftzuführungen sowie der Art der Gasdosierung können nur die Fehlergase bestimmt werden. Die Genauigkeit ist nur grob, da weder Temperatur- und Luftdruckmessungen erfolgen noch die Gassättigung des Öles nach der 3.Belüftung eingetreten sein muss. Diese Technik ist nicht für übersättigte Öle einsetzbar. Außerdem sind die speziellen Spritzen für den Probentransport ins Labor wenig geeignet.

Bei allen Analyseverfahren werden die Löslichkeitskoeffizienten der Gase im Öl entweder direkt zur Ergebnisbildung oder indirekt zur Festlegung der apparativen Parameter benötigt. Zu möglichen Bestimmungsverfahren gibt es keine besonderen Hinweise.

### Darstellung der Erfindung

### Technische Aufgabe

Aufgabe der Erfindung ist es, eine Headspace-Variante so zu entwickeln, dass eine einmalige Gleichgewichtseinstellung unter Einbeziehung atmosphärischer Luft die Vollanalyse der im Öl gelösten Gase, insbesondere auch die atmosphärischen Gase, im Labor bzw. in situ gestattet sowie durch eine einfache Modifizierung die Bestimmung von Löslichkeitskoeffizienten möglich ist.

### Technische Lösung

Erfindungsgemäß wird die Aufgabe durch die Merkmale der Ansprüche 1 und 11 gelöst. Vorteilhafte Ausgestaltungen ergeben sich aus den Unteransprüchen.

Das Verfahren ist durch folgende Schritte gekennzeichnet:
- vollständiges hermetisches Befüllen eines Gefäßes (1) bekannten Volumens mit Flüssigkeit im Durchfluss bis zum thermischen Gleichgewicht,
- Entfernen des Fülleinsatzes (2) bekannten Volumens ohne Flüssigkeitsverlust aus dem Gefäß (1), so dass im Gefäß (1) durch Einströmen von Umgebungsluft in das frei werdende Volumen ein anteiliger Gasraum (15) entsteht,
- wobei der anfängliche Inhalt des Gasraums (15) unter Einbeziehung der gemessenen Lufttemperatur und dem gemessenen Luftdruck mit dem Verschließen feststeht,
- hermetisches Verschließen des Gefäßes (1), um durch Kontaktieren von Flüssigkeit (14) und Gas das isotherme Lösungsgleichgewicht aller Gase herzustellen,
- Messung des Gleichgewichtsdrucks,
- definiertes isothermes Einführen von Luft oder Inertgas in den Gasraum (15) und
- anschließende Probennahme zur Gasraumanalyse.

Die anschließende Probennahme dient zur Bestimmung der Gehalte aller in der Flüssigkeit der Hochspannungsanlage gelösten Gase, korrigiert um die zugeführte Luft, und erfolgt nach dem Henry-Dalton'schen Gesetz.

Bei einer weiteren Ausführungsform erfolgt das Befüllen der Gewindeflasche mit entgaster Flüssigkeit. Das Zudosieren eines registrierten Eichgasvolumens erfolgt nach dem 1. isothermen Lösungsgleichgewicht mit nachfolgender Einstellung eines 2. isothermen Lösungsgleichgewichts vor der Inertgaszuführung. Die anschließende Probennahme zur Gasraumanalyse erfolgt für die Bestimmung der Löslichkeitskoeffizienten der Gase in der Flüssigkeit der Hochspannungsanlage nach dem Henry-Dalton'schen Gesetz.

Die Vorrichtung zur Bereitstellung der in Flüssigkeiten von Hochspannungsanlagen gelösten Gase für die externe Analyse besteht aus einem Gefäß bekannten Volumens mit einem Deckel mit Innenloch, einem zylindrischen Fülleinsatz bekannten Volumens, einem Kunststoffdeckel mit Septum, einem Handlocher und einem T-Verbinder.

Der zylindrische Fülleinsatz ist an einer Auflegscheibe mit Dichtring befestigt und im Bereich unterhalb der Auflegscheibe im Durchmesser stark verjüngt, so dass ein Ablaufstutzen mit Einweghahn von außen in die Auflegscheibe führt und unterhalb dieser frei im Gefäß endet. Der Fülleinsatz besitzt eine durchgehende Bohrung, die oberhalb der Auflegscheibe in den Zulaufstutzen mit Einweghahn übergeht. Mit dem Fülleinsatz ist ein Temperatursensor verbunden.

Der zylindrische Fülleinsatz reicht fast bis zum Boden des Gefäßes und passt bewegbar mit geringem Spiel in die Öffnung des Gefäßes.

Der Ablaufstutzen führt zu einem Sammelbehälter, die mit dem Flüssigkeitsraum des Ausdehnungsgefäßes der Hochspannungsanlage verbunden ist.

Der Temperatursensor ist etwa mittig im Fülleinsatz eingebracht und führt über ein Verbindungskabel nach außen zu einer Versorgungs- und Anzeigeeinheit.

Der Kunststoffdeckel, welcher nach dem Füllvorgang das Gefäß für Transport, Aufbewahrung, Extraktion und Gasbereitstellung verschließt, enthält eine Scheibendichtung als Septum und ist mit einem Sicherungsring ausgestattet.

Der Handlocher enthält einen Dorn, welcher abgestimmt ist auf die Dicke des Kunststoffdeckels ohne Septum.

Der T-Verbinder enthält ein Druckmessgerät, einen Einweghahn, der bei Bedarf mit einer Injektionsspritze verbunden ist, und eine Kanüle.

Das Gefäß wird in situ und/oder als Transport- und Aufbewahrungsgefäß genutzt.

Für die erfolgreiche Realisierung der Erfindung ist es vor allem von Bedeutung, dass für die Labor- und In Situ-Variante die Probenahme, der Transport bzw. die Aufbewahrung sowie die Gasextraktion zur Analyse in einem starren Gefäß stattfinden und dass die beiden Belüftungsschritte so gestaltet werden, dass diese ausreichend erfasst und korrigiert werden können.

### Vorteilhafte Wirkungen

Durch das erfindungsgemäße Verfahren und die Vorrichtung zur Durchführung des Verfahrens wird die Probennahme und deren Transport wesentlich vereinfacht. Die Gleichgewichtseinstellung erfolgt unter Einbeziehung atmosphärischer Luft. Die Vollanalyse der im Öl gelösten Gase, insbesondere auch die atmosphärischen Gase, kann im Labor bzw. in situ durchgeführt werden. Durch eine einfache Modifizierung ist die Bestimmung von Löslichkeitskoeffizienten möglich.

### Kurze Beschreibung der Zeichnungen

Die Erfindung soll nachfolgend anhand eines Ausführungsbeispiels zur Bestimmung der in der Flüssigkeit gelösten Gase näher erläutert werden. Vorzugsweise Anwendungen sind Öltransformatoren. Hierzu zeigen

Figur 1 die erfindungsgemäße Gewindeflasche mit Schraubdeckel und Fülleinsatz,

Figur 2 die befüllte Gewindeflasche mit Schraubdeckel,

Figur 3 ein Handlocher,

Figur 4 ein erfindungsgemäßer T-Verbinder und,

Figur 5 eine Injektionsspritze.

### Weg(e) zur Ausführung der Erfindung

Die Zeichnungen 1 bis 5 zeigen die erfindungsgemäße Vorrichtung als Labor- bzw. In Situ-Variante für die Bereitstellung der im Isolieröl gelösten Gase für die externe Vollanalyse.

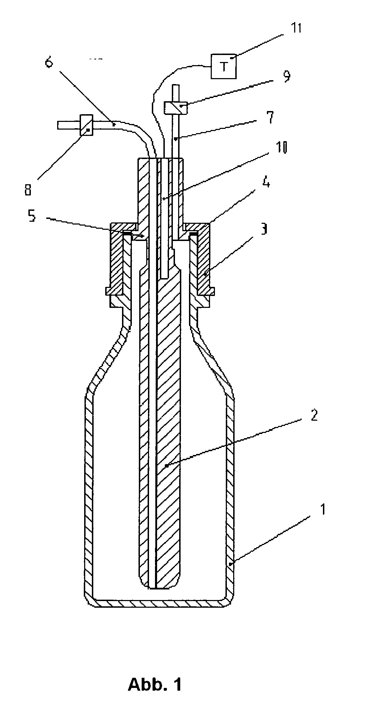

Die in der Fig. 1 gezeigte Vorrichtung besteht aus einer Gewindeflasche 1 bekannten Volumens, die wahlweise mit zwei verschiedenen Schraubdeckeln 3 bzw. 12 verschlossen werden kann. Ein Deckel mit Innenloch 3 umschließt bewegbar einen zylindrischen Fülleinsatz 2 bekannten Volumens, der an einer Auflegscheibe 5 mit Dichtring 4 befestigt ist und nur mit geringem Spiel in die Öffnung der Flasche 1 passt und fast bis zum Boden reicht. Der Fülleinsatz 2 besitzt eine durchgehende Bohrung, die oberhalb der Auflegscheibe 5 in den Zulaufstutzen 6 mit Einweghahn 8 übergeht. Ein Ablaufstutzen 7 mit Einweghahn 9 führt von außen senkrecht ebenfalls in die Auflegscheibe 5 und endet unterhalb dieser frei in das Gefäß, da hier der Fülleinsatz 2 im Durchmesser stark verjüngt ist. Etwa mittig im metallischen Fülleinsatz 2 ist ein Temperatursensor 10 eingebracht, von dem ein Verbindungskabel nach außen zur Versorgungs- und Anzeigeeinheit 11 führt.

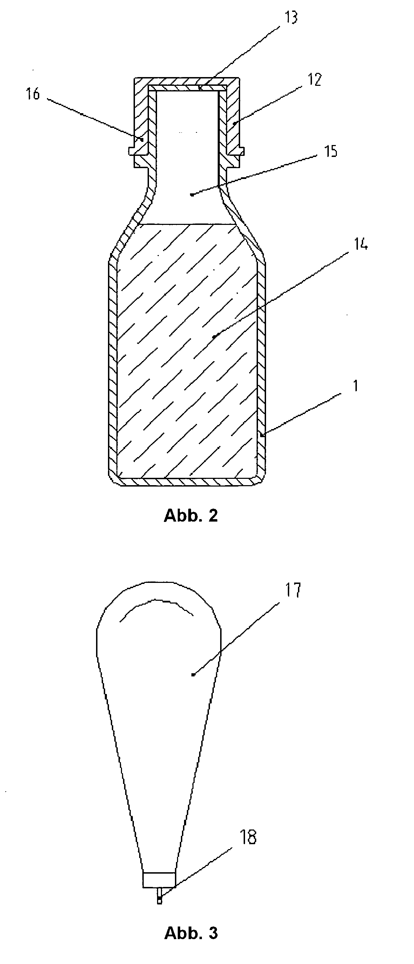

Der Deckel 12 in Fig. 2 ist aus Kunststoff und hat eine Scheibendichtung 13 eingelegt, die gleichzeitig als Septum geeignet ist. Der Deckel 12 ist mit einem Sicherungsring 16 versehen.

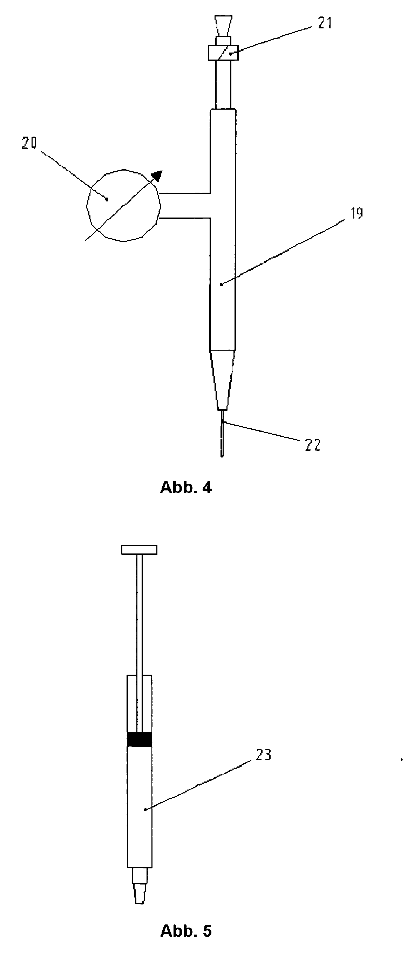

Die Vorrichtung zur Bereitstellung der im Öl gelösten Gase wird komplettiert durch einen Handlocher 17 (Fig. 3), der einen Dorn 18 besitzt, der auf die Dicke des Deckels ohne Septum abgestimmt ist sowie durch ein T-Verbinder 19 (Fig. 4), der ein Druckmessgerät 20, einen Einweghahn 21 und eine Kanüle 22 verbindet. In den Einweghahn 21 kann eine Injektionsspritze 23 (Fig. 5) eingeführt werden. Zum Befüllen wird die Flasche 1 der Vorrichtung senkrecht aufgestellt, der Fülleinsatz 2 bis zum Anschlag eingeführt und mit dem Deckel 3 dicht verschlossen. An den Zulaufstutzen 6 wird die Schlauchverbindung zum Probenahmestutzen des Transformators gesteckt und an den Ablaufstutzen 7 die Schlauchverbindung zum Sammelbehälter. Die Temperaturmessung 11 wird eingeschaltet. Die Einweghähne 8 bzw. 9 sind geöffnet.

Jetzt ist die Befüllvariante der Vorrichtung (Fig. 1) einsatzbereit. Am Transformator wird das Probenahmeventil geöffnet und das Öl gelangt über den Einfüllstutzen 6 in die Flasche 1. Der Ölspiegel steigt an, dabei verdrängt das Öl die Luft in der Flasche. Die letzte Luft entweicht, wenn das Öl unterhalb der Auflegscheibe 5 die Verjüngung des Fülleinsatzes 2 erreicht und über den Ablaufstutzen 7 zum Sammelbehälter gelangt. Während des Öldurchflusses wird die Temperaturmessung 11 beobachtet. Nach dem Anstieg der Temperatur beginnt diese zu stagnieren und wird registriert. In dieser Phase können die beiden Einweghähne 8 bzw. 9 geschlossen und die beiden selbstschließenden Schlauchverbinder von den Stutzen 6 und 7 entfernt werden.

Nun wird der Schraubdeckel 3 vollständig gelöst und der Fülleinsatz 2 langsam gezogen. Ist er vollständig aus dem Öl heraus, wird das Abtropfen des Öles abgewartet und dabei die Lufttemperatur unmittelbar über dem Öl an der Messeinrichtung erfasst. Der Fülleinsatz 2 wird vollständig herausgezogen, abgelegt und unmittelbar anschließend der Schraubdeckel 12 mit Dichtung bis zum Einrasten des Sicherungsrings 16 aufgebracht. Abschließend wird der Luftdruck erfasst.

Damit ist die Probenahme abgeschlossen. Die entnommene Ölprobe 14 ist repräsentativ und kann transportiert und aufbewahrt werden (Fig. 2).

Die Lage der Flasche 1 in dieser Zeit sowie die Lagerungstemperatur sind nicht vorgeschrieben. Vor der Gasraumanalyse im Labor wird die Flasche 1 mit einem Horizontalschüttler einzeln oder in einer Sendung von bis zu 12 Stück über mindestens 10 min. zur isothermen Gleichgewichtseinstellung bewegt. Danach wird die Flasche 1 senkrecht gestellt. Nach der Ölberuhigung wird mit dem Handlocher 17 der Plastedeckel mittig gelocht. Danach wird der T-Verbinder 19 mit der Kanüle 22 durch das Loch in das Septum 13 eingeführt und dies durchstochen. Sodann wird der Gleichgewichtsdruck registriert und dann der Einweghahn 21 geöffnet. Ist der Atmosphärendruck erreicht, wird ca. 1 cm³ Luft mit der auf den Einweghahn 21 aufgesetzten Injektionsspritze 23 zudosiert. Unmittelbar danach wird der Einweghahn 21 geschlossen und der Enddruck des Gasraumes 15 sowie die Raumtemperatur registriert und der T-Verbinder 19 mit der Kanüle 22 entfernt. Innerhalb der nächsten 30 Minuten sollten die Gasanalysen ausgeführt werden, die Probenahme erfolgt in bekannter Weise über das Septum 13.

Für die Anwendung zur Bestimmung der Löslichkeitskoeffizienten ist Voraussetzung, dass der beschriebene Befüllvorgang mit entgastem Öl stattfindet (Fig. 1). Nach der isothermen Gleichgewichtseinstellung (Fig. 2) wird der Gleichgewichtsdruck registriert und dann über Einweghahn 21 Eichgas (Fehlergas in Inertgas) zudosiert und der Druck gemessen. Die Flasche 1 wird nochmals analog im Horizontalschüttler bewegt. Danach wird die Flasche 1 wieder senkrecht gestellt und nach der Ölberuhigung der T-Verbinder 19 und Kanüle 22 über das Septum 13 eingeführt. Sodann erfolgt über den Einweghahn 21 die Zuführung von Inertgas bis zu einem leichten Überdruck, der registriert wird. Der T-Verbinder 19 mit Kanüle 22 wird entfernt. Innerhalb der nächsten 30 Minuten sollten die Gasanalysen ausgeführt sein, die Probenahme erfolgt in bekannter Weise über das Septum 13.

Mit den vorliegenden Analysenergebnissen der Konzentrationen der Gase im Gasraum 15 und der bekannten Zusammensetzung des Eichgases sowie den registrierten Angaben von dem 1. Gleichgewichtsdruck, dem Dosierdruck des Eichgases und dem Enddruck bei der Probenahme lassen sich nach dem Henry-Dalton'schen Gesetz der Gaslöslichkeit mit der Gleichgewichtstemperatur und den bekannten Abmessungen der Flasche 1 und des Fülleinsatzes 2 die Löslichkeitskoeffizienten für jedes Gas ermitteln. Ihre Werte gelten nur für die Gleichgewichtstemperatur und sind eventuell abhängig von der Ölsorte bzw. von dem Alterungszustand des Öles.

## Patentansprüche

1. Verfahren zur Bereitstellung der in Flüssigkeiten von Hochspannungsanlagen gelösten Gase für die externe Analyse auf Basis der Gleichgewichtsextraktion unter Einbeziehung atmosphärischer Luft, **gekennzeichnet durch**
die Schritte
- vollständiges hermetisches Befüllen eines Gefäßes (1) bekannten Volumens mit Flüssigkeit im Durchfluss bis zum thermischen Gleichgewicht,
- Entfernen eines Fülleinsatzes (2) bekannten Volumens ohne Flüssigkeitsverlust aus dem Gefäß (1), so dass im Gefäß (1) **durch** Einströmen von Umgebungsluft in das frei werdende Volumen ein anteiliger Gasraum (15) entsteht,
- wobei der anfängliche Inhalt des Gasraums (15) unter Einbeziehung der gemessenen Lufttemperatur und dem gemessenen Luftdruck mit dem Verschließen feststeht,
- hermetisches Verschließen des Gefäßes (1), um **durch** Kontaktieren von Flüssigkeit (14) und Gas das isotherme Lösungsgleichgewicht aller Gase herzustellen,
- Messung des Gleichgewichtsdrucks,
- definiertes isothermes Einführen von Luft oder Inertgas in den Gasraum (15) und
- anschließende Probennahme zur Gasraumanalyse.

2. Verfahren nach Anspruch 1, **dadurch gekennzeichnet, dass**
die anschließende Probennahme zur Bestimmung der Gehalte aller in der Flüssigkeit der Hochspannungsanlage gelösten Gase, korrigiert um die zugeführte Luft, dient und nach dem Henry-Dalton'schen Gesetz erfolgt.

3. Verfahren nach Anspruch 1, **dadurch gekennzeichnet, dass**
das Befüllen des Gefäßes (1) mit entgaster Flüssigkeit und das Zudosieren eines registrierten Eichgasvolumens nach dem 1. isothermen Lösungsgleichgewicht mit nachfolgender Einstellung eines 2. isothermen Lösungsgleichgewichts vor der Inertgaszuführung erfolgt.

4. Verfahren nach Anspruch 3, **dadurch gekennzeichnet, dass**
die anschließende Probennahme zur Gasraumanalyse für die Bestimmung der Löslichkeitskoeffizienten der Gase in der Flüssigkeit der Hochspannungsanlage nach dem Henry-Dalton'schen Gesetz erfolgt.

5. Verfahren nach Anspruch 1 oder 2, **dadurch gekennzeichnet, dass**
die Luft messbar vom Gleichgewichtsdruck bis zum Atmosphärendruck oder einem höheren Druck zugeführt wird.

6. Verfahren nach Anspruch 1 oder 2, **dadurch gekennzeichnet, dass**
bei einem Gleichgewichtsdruck gleich oder größer dem Atmosphärendruck keine zusätzliche Luftzufuhr erfolgt.

7. Verfahren nach Anspruch 1 oder 2, **dadurch gekennzeichnet, dass**
nach der Einstellung des isothermen Lösungsgleichgewichts und vor der Probenahme zur Gasraumanalyse isotherm ein Inertgas messbar vom Gleichgewichtsdruck bis zum Atmosphärendruck oder einem höheren Druck in den Gasraum (15) eingeführt wird.

8. Verfahren nach Anspruch 1 oder 7, **dadurch gekennzeichnet, dass**
bei gasungesättigten Flüssigkeiten ohne Messung des Gleichgewichtsdruckes Inertgas bis zum Atmosphärendruck in den Gasraum (15) eingeführt wird.

9. Verfahren nach Anspruch 1 oder 7, **dadurch gekennzeichnet, dass**
bei gasungesättigten Flüssigkeiten ohne Messung des Gleichgewichtsdruckes Luft bis zum Atmosphärendruck in den Gasraum (15) eingeführt wird, um abschließend nach dem Henry-Dalton'schen Gesetz die Gehalte der in der Flüssigkeit der Hochspannungsanlage gelösten Fehlergase zu bestimmen.

10. Verfahren nach einem der Ansprüche 1 bis 9, **dadurch gekennzeichnet, dass** die Kontaktierung bis zum isothermen Gleichgewicht von Flüssigkeit (14) und Gas im Gefäß durch manuelles oder maschinelles Schütteln erfolgt.

11. Vorrichtung zur Bereitstellung der in Flüssigkeiten von Hochspannungsanlagen gelösten Gase für die externe Analyse **dadurch gekennzeichnet, dass**
- ein Gefäß (1) bekannten Volumens mit einem Deckel mit Innenloch (3),
- ein zylindrischen Fülleinsatz (2) bekannten Volumens,
- ein Kunststoffdeckel (12),
- ein Handlocher (17) und
- ein T-Verbinder (19) bereitgestellt werden.

12. Vorrichtung nach Anspruch 11 **dadurch gekennzeichnet, dass**
der zylindrische Fülleinsatz (2) an einer Auflegscheibe (5) mit Dichtring (4) befestigt und im Bereich unterhalb der Auflegscheibe (5) im Durchmesser stark verjüngt ist, so dass ein Ablaufstutzen (7) mit Einweghahn (9) von außen in die Auflegscheibe (5) führt und unterhalb dieser frei im Gefäß (1) endet, der Fülleinsatz (2) eine durchgehende Bohrung besitzt, die oberhalb der Auflegscheibe (5) in den Zulaufstutzen (6) mit Einweghahn (8) übergeht und ein Temperatursensor (10) mit dem Fülleinsatz (2) verbunden ist.

13. Vorrichtung nach Anspruch 11 oder 12 **dadurch gekennzeichnet, dass**
der zylindrische Fülleinsatz (2) fast bis zum Boden des Gefäßes (1) reicht und bewegbar mit geringem Spiel in die Öffnung des Gefäßes (1) passt.

14. Vorrichtung nach Anspruch 12 **dadurch gekennzeichnet, dass**
der Ablaufstutzen (7) zu einem Sammelbehälter führt, die mit dem Flüssigkeitsraum des Ausdehnungsgefäßes der Hochspannungsanlage verbunden ist.

15. Vorrichtung nach Anspruch 12 **dadurch gekennzeichnet, dass**
der Temperatursensor (10) etwa mittig im Fülleinsatz (2) eingebracht ist und über ein Verbindungskabel nach außen zu einer Versorgungs- und Anzeigeeinheit führt.

16. Vorrichtung nach Anspruch 11 **dadurch gekennzeichnet, dass**
der Kunststoffdeckel (12), welcher nach dem Füllvorgang das Gefäß (1) für Transport, Aufbewahrung, Extraktion und Gasbereitstellung verschließt, eine Scheibendichtung (13) als Septum enthält und mit einem Sicherungsring (16) ausgestattet ist.

17. Vorrichtung nach Anspruch 11 **dadurch gekennzeichnet, dass**
der Handlocher (17) einen Dorn (18), welcher abgestimmt ist auf die Dicke des Kunststoffdeckels (12) ohne Septum, enthält.

18. Vorrichtung nach Anspruch 11 **dadurch gekennzeichnet, dass**
der T-Verbinder (19) ein Druckmessgerät (20), einen Einweghahn (21), der bei Bedarf mit einer Injektionsspritze (23) verbunden ist, und eine Kanüle (22) enthält.

## Claims

1. A method for providing gases dissolved in liquids of high-voltage facilities for the external analysis based on the equilibrium type extraction using ambient air, **characterized by** the steps of
- hermetically filling a receptacle (1) of known volume completely with a liquid during flow up to the thermal equilibrium;
- removing a filling insert (2) of known volume without any ullage out of said receptacle (1) such that within said receptacle (1) a proportionate gas chamber (15) is resulting from the inflow of ambient air into said volume to be released;
- wherein the initial content of said gas chamber (15) using the measured air temperature and the measured atmospheric pressure is fixed with closing;
- hermetically sealing said receptacle (1) in order to provide the isothermal solution equilibrium of all of the gases by means of contacting said liquid (14) and gas;
- measurement of the equilibrium pressure;
- defined isothermal introducing of air or inert gas into said gas chamber (15); and
- thereafter sampling for the gas chamber analysis.

2. A method according to claim 1, **characterized in that** said subsequent sampling is contributing to the determination of the contents of all of said gases dissolved in the liquid of said high-voltage facility, corrected by the supplied air, and is carried out in accordance with Dalton law of partial pressures.

3. A method according to claim 1, **characterized in that** filling said receptacle (1) with degassed liquid and metering a recorded calibration gas volume after the first isothermal solution equilibrium with the subsequent adjustment of a second isothermal solution equilibrium is carried out prior to the inert gas supply.

4. A method according to claim 3, **characterized in that** said subsequent sampling for said gas chamber analysis is carried out in accordance with Dalton law of partial pressures for the determination of the solubility coefficients of said gases within the liquid of said high-voltage facility.

5. A method according to claim 1 or 2, **characterized in that** said air is measurably supplied from said equilibrium pressure up to said atmospheric pressure or a higher pressure.

6. A method according to claim 1 or 2, **characterized in that** said no additional air supply is carried out at an equilibrium pressure greater than or equal to said atmospheric pressure.

7. A method according to claim 1 or 2, **characterized in that** after said adjustment of the isothermal solution equilibrium and prior to said sampling for said gas chamber analysis an inert gas is measurably introduced from said equilibrium pressure up to said atmospheric pressure or a higher pressure into said gas chamber (15).

8. A method according to claim 1 or 7, **characterized in that** with liquids being unsaturated of gas said inert gas is introduced without any measurement of said equilibrium pressure up to said atmospheric pressure into said gas chamber (15).

9. A method according to claim 1 or 7, **characterized in that** with liquids being unsaturated of gas, air is introduced without any measurement of the equilibrium pressure up to the atmospheric pressure into said gas chamber (15) in order to conclusively determine in accordance with Dalton law of partial pressures the contents of the fault gases dissolved in the liquid of said high-voltage facility.

10. A method according to any one of claims 1 to 9, **characterized in that** contacting up to the isothermal equilibrium of said liquid (14) and gas is carried out by shaking manually or mechanically in said receptacle.

11. An apparatus for providing said gases dissolved in liquids of high-voltage facilities for the external analysis, **characterized in that** are provided
- a receptacle (1) of known volume with a cover having an centre hole (3);
- a cylindrical filling insert (2) of known volume;
- a plastic cover (12);
- a manual punch (17); and
- a T-joint (19).

12. An apparatus according to claim 11, **characterized in that** said cylindrical filling insert (2) is attached to a gasket (5) having a sealing ring (4), and is highly tapered diametrically in the region beneath said gasket (5) such that a discharge neck (7) having a single-bore stopcock (9) is conducted into said gasket (5) from the outside, and is clearly terminating beneath thereof within said receptacle (1), wherein said filling insert (2) has a through bore which is passing upstream of said gasket (5) into the inlet pipe (6) having a single-bore stopcock (8), and a temperature sensor (10) is connected to said filling insert (2).

13. An apparatus according to claim 11 or 12, **characterized in that** said cylindrical filling insert (2) is almost reaching to the bottom of said receptacle (1), and is movably fitting with low mechanical play into the aperture of said receptacle (1).

14. An apparatus according to claim 12, **characterized in that** said discharge neck (7) is conducted to a receiver tank which is connected to said liquid space of the conservator of said high-voltage facility.

15. An apparatus according to claim 12, **characterized in that** said temperature sensor (10) is inserted in said filling insert (2) approximately centrally, and is leading to the outside toward a supply and display unit via a connecting cable.

16. An apparatus according to claim 11, **characterized in that** said plastic cover (12), which after the filling action is locking said receptacle (1) for the transport, storage, extraction and provision of gas, includes a disk type gasket (13) as a septum and is equipped with a retaining ring (16).

17. An apparatus according to claim 11, **characterized in that** said manual punch (17) includes a spike (18) which is adapted ton the thickness of said plastic cover (12) without having any septum.

18. An apparatus according to claim 11, **characterized in that** said T-joint (19) comprises a pressure gauge (20), a single-bore stopcock (21) which is connected to a hypodermic syringe (23), if required, and a tubule (22).

## Revendications

1. Procédé pour la réalisation des gazes en solution dans les liquides des installations à haute tension pour l'analyse externe sur la base de la extraction d'équilibre en fermant intégrant l'air atmosphérique, ledit procédé étant **caractérisé par** les pas de
- verser hermétiquement d'un liquide entièrement dans un récipient (1) de volume connu, en passent jusqu'au équilibre thermique;
- enlever d'un insert de remplissage (2) d'un volume connu à ce récipient (1) sans perte de liquide de manière que dans le récipient (1) il en résulte un espace de gaz (15) proportionnel à cause de l'air ambiant étant rentrant dans le volume se dégageant;
- ledit contenu premier d'espace de gaz (15) est déterminé en fermant intégrant la température de l'air mesurée et la pression de l'air mesurée en fermant;
- verser hermétiquement du récipient (1) pour etablir l'équilibre de solution isotherme de tous les gazes par mettre un contact du liquide (14) et de gaz;
- le mesurage de la pression d'équilibre;
- l'introduction définie isotherme de l'air ou gaz inerte dans l'espace de gaz (15); et
- l'échantillonnage suivant pour l'analyse de l'espace de gaz.

2. Procédé selon la revendication 1, **caractérisé en ce que** ledit échantillonnage suivant sert pour la destination de teneurs de tous les gazes en solution dans le liquide de l'installation à haute tension, corrigé de l'air amené, et il s'est effectué conforme au loi de Dalton.

3. Procédé selon la revendication 1, **caractérisé en ce que** le remplissage du récipient (1) avec du liquide dégazé et le dosage avec un volume registré de l'étalon de gaz sont effectués après le premier équilibre de solution isotherme avec un réglage suivant d'un deuxième équilibre de solution isotherme avant l'amenée de gaz inerte.

4. Procédé selon la revendication 3, **caractérisé en ce que** ledit échantillonnage suivant ayant lieu à l'analyse de l'espace de gaz pour la destination des coefficients de solubilité des gazes dans le liquide de l'installation à haute tension, conforme au loi de Dalton.

5. Procédé selon les revendications 1 ou 2, **caractérisé en ce que** l'air est alimenté mesurablement de la pression d'équilibre à la pression atmosphérique ou une pression plus élevé.

6. Procédé selon les revendications 1 ou 2, **caractérisé en ce que** en cas d'une pression d'équilibre égal ou supérieur que la pression atmosphérique il n'a pas lieu une amenée supplémentaire de l'air.

7. Procédé selon les revendications 1 ou 2, **caractérisé en ce que** après le réglage de l'équilibre de solution isotherme et avant ledit échantillonnage pour l'analyse de l'espace de gaz, un gaz inerte est introduit dans l'espace de gaz (15) étant mesurable de la pression d'équilibre à la pression atmosphérique ou une pression plus élevé.

8. Procédé selon les revendications 1 ou 7, **caractérisé en ce que** en cas de liquides étant non saturé de gaz, sans mesurage de la pression d'équilibre, le gaz inerte est introduit dans l'espace de gaz (15) jusqu'à la pression atmosphérique.

9. Procédé selon les revendications 1 ou 7, **caractérisé en ce que** en cas de liquides étant non saturé de gaz, sans mesurage de la pression d'équilibre, l'air est introduit dans l'espace de gaz (15) jusqu'à la pression atmosphérique pour déterminer finalement les teneurs de gazes défectueux étant dissous dans le liquide de l'installation à haute tension, conforme au loi de Dalton.

10. Procédé selon l'une des revendications 1 à 9, **caractérisé en ce que** l'établissement du contact jusqu'au équilibre isotherme du liquide (14) et de gaz a lieu par agiter manuellement ou mécaniquement.

11. Dispositif pour la réalisation des gazes en solution dans les liquides des installations à haute tension pour l'analyse externe, **caractérisé en ce que**
- un récipient (1) de volume connu avec un couvercle à trou intérieur (3),
- un insert de remplissage cylindrique (2) de volume connu,
- un couvercle de plastique (12),
- une perforateur à main (17), et
- un connecteur type T (19)
sont préparés.

12. Dispositif selon la revendication 11, **caractérisé en ce que** l'insert de remplissage cylindrique (2) est attaché à l'un disque de butée (5) avec la bague d'étanchéité (4) et il s'amincit fortement en diamètre dans la région au-dessous ledit disque de butée (5) de manière qu'une tubulure de décharge (7) avec un robinet à passage direct (9) mène dans le disque de butée (5) du dehors et termine librement au-dessous de celle-ci dans le récipient (1), ledit insert de remplissage (2) possède un trou de passage lequel passe à la tubulure de remplissage (6) avec le robinet à passage direct (8) au-dessus de disque de butée (5), et un détecteur de température (10) est relié avec l'insert de remplissage (2).

13. Dispositif selon les revendications 11 ou 12, **caractérisé en ce que** l'insert de remplissage cylindrique (2) s'étend presque jusqu'au fond de récipient (1), et va mobilement avec peu de tolérance dans la ouverture de récipient (1).

14. Dispositif selon la revendication 12, **caractérisé en ce que** la tubulure de décharge 7 mène à l'un récipient collecteur, lequel est relié avec l'espace de liquide du récipient d'expansion dans l'installation à haute tension.

15. Dispositif selon la revendication 12, **caractérisé en ce que** le détecteur de température (10) est inséré environ au milieu dans ledit insert de remplissage (2), et mène par un câble de connexion vers l'extérieur à une unité d'alimentation et de visualisation.

16. Dispositif selon la revendication 11, **caractérisé en ce que** le couvercle de plastique (12) lequel fermé le récipient (1) après le remplissage pour le transport, la conservation, l'extraction et pour mettre gaz à la disposition, comprend un joint plat (13) en tant qu'un septum et est équipé d'une bague d'arrêt (16).

17. Dispositif selon la revendication 11, **caractérisé en ce que** la perforateur à main (17) comprend une épine en métal (18) laquelle est harmonisé à l'épaisseur couvercle de plastique (12) sans septum.

18. Dispositif selon la revendication 11, **caractérisé en ce que** le connecteur type T (19) comprend un instrument de mesure de la pression (20), un robinet à passage direct (21) lequel en cas de besoin est relié avec une seringue hypodermique (23), et une canule (22).
